## Europäisches Patentamt

## European Patent Office

## Office européen des brevets

(19)

(11) Veröffentlichungsnummer: **0 029 196**
**B1**

(12)

# EUROPÄISCHE PATENTSCHRIFT

(45) Veröffentlichungstag der Patentschrift:
21.09.83

(51) Int. Cl.³: **B 22 C 1/18,** C 04 B 19/04,
C 07 H 15/12

(21) Anmeldenummer: 80106920.4

(22) Anmeldetag: 10.11.80

(54) Bindemittel auf Basis von Alkalimetallsilikatlösungen und deren Verwendung.

(30) Priorität: 17.11.79 DE 2946500

(43) Veröffentlichungstag der Anmeldung:
27.05.81 Patentblatt 81/21

(45) Bekanntmachung des Hinweises auf die Patenterteilung:
21.09.83 Patentblatt 83/38

(84) Benannte Vertragsstaaten:
DE GB NL SE

(56) Entgegenhaltungen:
DE-A-2 117 774
DE-A-2 641 249
DE-A-2 714 889
DE-A-2 750 294
DE-B-1 028 265
FR-A-1 180 881
US-A-2 016 962
US-A-2 181 929

(73) Patentinhaber: Henkel Kommanditgesellschaft auf Aktien, Postfach 1100 Henkelstrasse 67,
D-4000 Düsseldorf 1 (DE)

(72) Erfinder: Friedemann, Wolfgang, Dr.,
Stephanusstrasse 14, D-4040 Neuss (DE)
Erfinder: Maak, Norbert, Dr., Liebigstrasse 18,
D-4040 Neuss (DE)
Erfinder: Feulner, Kurt, Heibauerfeld 10, D-4300 Essen
(DE)

Bindemittel auf Basis von Alkalimetallsilikatlösungen und deren Verwendung

Gegenstand der Erfindung sind Bindemittelzusammensetzungen auf der Basis wässriger Alkalimetallsilikatlösungen, die — bedingt durch einen Zusatz organischer Verbindungen — verbesserte anwendungstechnische Eigenschaften aufweisen. Gegenstand der Erfindung ist ferner die Verwendung derartiger Bindemittel zur Herstellung von Formkörpern, insbesondere von Formen und Kernen für den Metallguss.

Bindemittelzusammensetzungen auf Basis wässriger Alkalimetallsilikatlösungen sind seit langem bekannt und werden in grossem Umfang in der Praxis eingesetzt. Die hierzu verwendeten Alkalimetallsilikatlösungen — insbesondere Natriumsilikatlösungen — weisen in der Regel ein Molverhältnis $SiO_2:Me_2O$ (Me = Alkalimetall) im Bereich von 2,0 bis 3,4 : 1 sowie einen Feststoffgehalt von 35 bis 50 Gewichtsprozent auf. Solche Bindemittelzusammensetzungen dienen beispielsweise zur Herstellung von mineralischen Isolierstoffen, Imprägniermitteln, Überzugsmassen, Anstrichmitteln und Kitten sowie zum Verkleben von Holz, Papier, Keramik und mineralischem Material.

Auch zur Herstellung von Formkörpern für den Metallguss lassen sich derartige Bindemittel verwenden. Üblicherweise werden hierbei die wässrige Alkalimetallsilikatlösung mit einem feinteiligen Füllstoff — beispielsweise Sand — vermischt, die resultierende Mischung in die gewünschte Form gebracht und mit Hilfe geeigneter Härter verfestigt. Geeignete Härter für diesen Zweck sind zum Beispiel Kohlendioxidgas beziehungsweise sauer reagierende oder Säure abspaltende Verbindungen. Ein wesentlicher Nachteil dieses Verfahrens ist darin zu sehen, dass sich die fertigen Formkörper, dass heisst Formen und Kerne, nach dem Metallguss nur äusserst schwer zerbrechen und von den erstarrten Gussteilen trennen lassen. Um den Zerfall der Formkörper nach erfolgtem Guss zu fördern, werden den Bindemitteln üblicherweise verschiedene Kohlenhydrate, wie Zucker, Melasse, Stärke od. Stärke- beziehungsweise Cellulosederivate zugefügt. Diese Zusätze, die in Mengen bis zu 10 Gewichtsprozent in der Bindemittelzusammensetzung enthalten sein können, führen jedoch meist zu einer unerwünschten Wasseraufnahme der gehärteten Formkörper während der Lagerung. Dies bedingt eine wesentliche Verminderung der Festigkeit der Formkörper, insbesondere im Bereich deren Kanten.

Die Aufgabe der vorliegenden Erfindung war es daher, ein Bindemittel auf Basis wässriger Alkalimetallsilikatlösungen zu entwickeln, das insbesondere zur Herstellung von Formkörpern für den Metallguss geeignet ist, die vorstehend aufgezeigten Nachteile jedoch nicht aufweist.

Gegenstand der Erfindung sind somit Bindemittel auf der Basis wässriger Alkalimetallsilikatlösungen, die ein Molverhältnis $SiO_2:Me_2O$ im Bereich von 2,0 bis 3,4 : 1, wobei Me ein Alkalimetall bedeutet, und einen Feststoffgehalt von 35 bis 50 Gewichtsprozent aufweisen, die dadurch gekennzeichnet sind, dass sie reduktiv aminierte Mono-, Di- oder Oligosaccharide enthalten.

Überraschenderweise wurde nämlich gefunden, dass ein Zusatz von reduktiv aminierten Sacchariden zum Bindemittel dessen anwendungstechnische Eigenschaften bei der Herstellung von Formkörpern wesentlich verbessert. Dies äussert sich einerseits in einer stark verminderten Hygroskopizität der gehärteten Formkörper sowie andererseits in einem leichteren Zerfall der zum Guss benutzten Formteile. Aufgrund der geringeren Hygroskopizität der Formkörper bleibt deren hohe Druckfestigkeit — insbesondere im Bereich der Kanten — auch während einer Lagerung bei Raumtemperatur über längere Zeit erhalten. Ferner zeigt sich, dass selbst bei kurzen Härtungszeiten der Formteile durch Begasen mit Kohlendioxid bereits gute Anfangsdruckfestigkeiten resultieren. Somit werden sowohl Handhabung und Transport der gehärteten Formkörper als auch deren Entfernung vom fertigen Gussstück wesentlich erleichtert.

Reduktiv aminierte Mono-, Di- oder Oligosaccharide im Sinne der Erfindung sind Umsetzungsprodukte entsprechender Saccharide beziehungsweise Saccharid-Gemische mit gebräuchlichen Aminierungskomponenten, die unter der Einwirkung von Wasserstoff gebildet werden. Als Aminierungskomponente für derartige Umsetzungsprodukte kommen neben Ammoniak primäre und sekundäre Amine in Frage sowie gleichfalls Gemische solcher Verbindungen. Geeignete Aminierungskomponenten sind beispielsweise Methylamin, Ethylamin, Butylamin, Ethanolamin, Dimethylamin, Diethylamin, Diethanolamin und Ammoniak. Zu den Sacchariden, die solche Umsetzungsprodukte zu bilden vermögen, zählen neben den Monosacchariden alle reduzierend wirkenden Di- und Oligosaccharide, zum Beispiel Glucose, Galaktose, Maltose, Lactose, Cellobiose, Maltotriose, Maltodextrine sowie sonstige reduzierend wirkende oligomere Stärkeabbauprodukte wie Glucosesirup.

Man erhält die erfindungsgemässen Umsetzungsprodukte — kurz Aminosaccharide genannt — beispielsweise durch Reaktion reduzierender Saccharide mit der Aminierungskomponente in wässrigem Medium und in Gegenwart von Wasserstoff sowie eines gebräuchlichen Hydrierungskatalysators in einem Druckgefäss. Ein entsprechendes Verfahren zur Herstellung solcher Produkte ist zum Beispiel in der US-A 2 016 962 beschrieben. Die Isolierung der gebildeten Aminosaccharide erübrigt sich im allgemeinen, da sich diese beispielsweise in Form einer 30-gewichtsprozentigen wässrigen Lösung im erfindungsgemässen Sinne einsetzen lassen.

Grundlage für die in den erfindungsgemässen Bindemitteln zu verwendenden Alkalimetallsilikatlösungen sind in der Regel handelsübliche Wasserglaslösungen, deren Molverhältnisse und Feststoffgehalte im Rahmen der vorstehend angegebenen Werte liegen. Entsprechende wässrige Alkalimetallsilikatlösungen können jedoch auch durch Auflösen pulverförmiger löslicher Alkalimetallwassergläser erhalten werden. Im Interesse eines guten Bindevermögens

enthalten die erfindungsgemässen Bindemittel bevorzugt ein Alkalimetallsilikat mit einem Molverhältnis $SiO_2:Me_2O$ im Bereich von 2,2 bis 2,6 : 1, wobei Me Natrium und/oder Kalium bedeutet.

Mithin können für die erfindungsgemässen Bindemittel ausser Natronwassergläsern auch Kaliwassergläser sowie entsprechende Mischgläser Verwendung finden.

Bindemittel gemäss der Erfindung enthalten in der Regel 0,1 bis 10 Gewichtsprozent, bezogen auf die gesamte Bindemittelzusammensetzung, eines reduktiv aminierten Mono-, Di- oder Oligosaccharids, wobei man im allgemeinen das Aminosaccharid dem Bindemittel in Form einer wässrigen Lösung zufügt. Bevorzugt sind Zusätze an Aminosaccharid im Bereich von 0,5 bis 5 Gewichtsprozent, bezogen auf die gesamte Bindemittelzusammensetzung, da sich mit derartigen Mengen hinreichend gute anwendungstechnische Effekte bei der Herstellung von Formkörpern erzielen lassen. Als reduktiv aminiertes Mono-, Di- oder Oligosaccharid kommt hierbei vorzugsweise ein unter Einwirkung von Wasserstoff erhaltenes Umsetzungsprodukt von Glucose, Galaktose oder Maltose, beziehungsweise deren Gemischen, mit Methylamin, Ethylamin, Butylamin oder Ethanolamin, beziehungsweise deren Gemischen, in Frage.

Im Hinblick auf eine verminderte Hygroskopizität der Formkörper hat sich ferner ein geringer Zusatz von Kaliummetaborat zum Bindemittel als vorteilhaft erwiesen. Dementsprechend können die erfindungsgemässen Bindemittel 0,1 bis 2 Gewichtsprozent $B_2O_3$, bezogen auf die gesamte Bindemittelzusammensetzung, in Form von Kaliummetaborat enthalten. Zweckmässigerweise fügt man das Kaliummetaborat — das zum Beispiel durch Vermischen von Borsäure mit der stöchiometrisch erforderlichen Menge an konzentrierter Kaliumhydroxidlösung erhalten werden kann — dem Bindemittel gleichfalls in Form einer wässrigen Lösung zu.

Wie vorstehend bereits ausgeführt, können die erfindungsgemässen Bindemittel mit Vorteil zur Herstellung von Formkörpern aus feinteiligen Füllstoffen, insbesondere von Formen und/oder Kernen für den Metallguss, Verwendung finden. Die Herstellung solcher Formkörper erfolgt in an sich bekannter Weise: Im allgemeinen werden 3 bis 6 Gewichtsprozent des erfindungsgemässen Bindemittels mit einem feinteiligen Füllstoff — üblicherweise Quarzsand — intensiv vermischt und unter Zusatz eines geeigneten Härters ausgehärtet. Als solche kommen alle für diesen Zweck gebräuchlichen anorganischen und/oder organischen Härter in Frage, beispielsweise Kohlendioxidgas, Ester mehrwertiger Alkohole wie Diacetin oder Triacetin, Ester der Bernsteinsäure, Glutarsäure und Adipinsäure.

Darüber hinaus lassen sich die erfindungsgemässen Bindemittel jedoch gleichfalls für alle diejenigen Zwecke verwenden, für die Bindemittel auf Basis wässriger Alkalimetallsilikatlösungen üblicherweise eingesetzt werden.

### Beispiele

In den folgenden Beispielen werden Bindemittel im Sinne der Erfindung sowie deren Verwendung näher erläutert.

### Beispiel 1

Das verwendete Bindemittel weist die folgende Zusammensetzung auf:

90 Gew.-% Natronwasserglas mit einem Molverhältnis $SiO_2:Na_2O$ von 2,48 : 1 und einem Feststoffgehalt von 47 Gew.-%

10 Gew.-% einer 30-gew.-%igen wässrigen Lösung von Methylglucamin (Umsetzungsprodukt von Methylamin mit Glucose unter Einwirkung von Wasserstoff).

Das Bindemittel wird erhalten durch Vermischen der wässrigen Lösungen.

Die Verwendung des Bindemittels zur Herstellung von Formkörpern wird in der nachstehend beschriebenen Weise durchgeführt:

100 Gewichtsteile Quarzsand (Körnung H34) werden mit 4 Gewichtsteilen des Bindemittels zwei Minuten lang intensiv gemischt. Anschliessend werden 170 g des Gemisches in einem Rammapparat nach Georg Fischer zu zylindrischen Kernen mit einer Höhe von 60 mm und einem Durchmesser von 50 mm geformt und verdichtet. Die erhaltene Kerne werden sodann durch Begasen mit Kohlendioxid (5 Sekunden bei 25°C und 1,5 bar Leitungsdruck) gehärtet. Unmittelbar darauf wird der Anfangswert der Druckfestigkeit der Kerne mit Hilfe eines Druckfestigkeitsprüfgerätes nach Georg Fischer ermittelt; der erhaltene Wert ist in Tabelle 1 wiedergegeben. Weitere Bestimmungen der Druckfestigkeit erfolgen nach einer Lagerung über 3 Tage bei Raumtemperatur und einer relativen Luftfeuchtigkeit von 50% sowie nach der gleichen Lagerdauer und anschliessender Temperung der Probekörper bei 900°C über zwei Minuten; die gemessenen Werte finden sich gleichfalls in Tabelle 1. Die Hygroskopizität der Probekörper wird bestimmt über die prozentuale Gewichtszunahme derselben nach einer Lagerdauer von 8 Tagen bei Raumtemperatur in einem Exsikkator über destilliertem Wasser (relative Luftfeuchtigkeit ca. 98%); Tabelle 1 zeigt die ermittelten Daten. Die Kantenfestigkeit der Prüfkörper — Anfangswert sowie nach dreitägiger Lagerung bei Raumtemperatur — wird manuell bestimmt.

Die nachfolgenden Beispiele entsprechen — soweit nicht anders vermerkt — den Angaben in Beispiel 1.

### Beispiel 2

Das verwendete Bindemittel enthält — abweichend von Beispiel 1 — als Aminosaccharid 3 Gew.-% Methylmaltamin (Umsetzungsprodukt von Methylamin mit Maltose unter Einwirkung von Wasserstoff). Die mit diesem Bindemittel bei der Herstellung von Formkörpern erzielten Ergebnisse sind in Tabelle 1 zusammengefasst.

## Beispiel 3

Das verwendete Bindemittel enthält — abweichend von Beispiel 1 — als Aminosaccharid 3 Gew.-% n-Butylglucamin (Umsetzungsprodukt von n-Butylamin mit Glucose unter Einwirkung von Wasserstoff); Ergebnisse siehe Tabelle 1.

## Beispiel 4

Das verwendete Bindemittel enthält — abweichend von Beispiel 1 — als Aminosaccharid 3 Gew.-% eines Umsetzungsproduktes von Glucosesirup mit Ethylamin und Wasserstoff; Ergebnisse siehe Tabelle 1.

## Beispiele 5 bis 9

Einem Bindemittel analog Beispiel 1 werden zusätzlich 1 Gew.-% $B_2O_3$, bezogen auf die gesamte Bindemittelzusammensetzung, in Form einer wässrigen Kaliummetaboratlösung zugesetzt. Bei der Herstellung der Formkörper wird ferner die Härtungsdauer der Probekörper durch Begasen mit Kohlendioxid variiert:
Beispiel 5: Begasungsdauer 5 Sekunden
Beispiel 6: Begasungsdauer 4 Sekunden
Beispiel 7: Begasungsdauer 3 Sekunden
Beispiel 8: Begasungsdauer 2 Sekunden
Beispiel 9: Begasungsdauer 1 Sekunde.
Ergebnisse siehe Tabelle 1.

## Beispiel 10

Das verwendete Bindemittel enthält — abweichend von Beispiel 1 — als Aminosaccharid 0,1 Gew.-% Methylgalaktamin (Umsetzungsprodukt von Methylamin mit Galaktose unter Einwirkung von Wasserstoff); Ergebnisse siehe Tabelle 1.

## Beispiel 11

Das verwendete Bindemittel enthält — abweichend von Beispiel 1 — als Aminosaccharid 5 Gew.-% Hydroxyethylglucamin (Umsetzungsprodukt von Ethanolamin mit Glucose unter Einwirkung von Wasserstoff); Ergebnisse siehe Tabelle 1.

## Beispiel 12

Das verwendete Bindemittel enthält — abweichend von Beispiel 1 — als Aminosaccharid 10 Gew.-% Ethylglucamin (Umsetzungsprodukt von Ethylamin mit Glucose unter Einwirkung von Wasserstoff); Ergebnisse siehe Tabelle 1.

## Beispiel 13

Das verwendete Bindemittel enthält — abweichend von Beispiel 1 — als Aminosaccharid 3 Gew.-% eines Umsetzungsproduktes von Maltodextrin mit Methylamin; Ergebnisse siehe Tabelle 1.

## Beispiel 14

Das verwendete Bindemittel enthält analog Beispiel 1 Methylglucamin als Aminosaccharid, jedoch in einer Zusatzmenge von 0,5 Gew.-%; Ergebnisse siehe Tabelle 1.

## Beispiel 15

Das verwendete Bindemittel weist die folgende Zusammensetzung auf:
90 Gew.-% Natronwasserglas mit einem Molverhältnis $SiO_2:Na_2O$ von 2,6 : 1 und einem Feststoffgehalt von 41,5 Gew.-%
10 Gew.-% einer 30-gewichtsprozentigen wässrigen Lösung von Methylglucamin analog Beispiel 1.
Ergebnisse siehe Tabelle 1.

## Beispiel 16

Das verwendete Bindemittel weist die folgende Zusammensetzung auf:
90 Gew.-% eines Natrium/Kalium-Mischglases mit einem Gesamtmolverhältnis $SiO_2:Me_2O$ von 2,6 : 1, gleichen Anteilen Natron- und Kaliwasserglas und einem Feststoffgehalt von 39,7 Gew.-%
10 Gew.-% einer 30-gewichtsprozentigen wässrigen Lösung von Methylglucamin analog Beispiel 1.
Ergebnisse siehe Tabelle 1.

## Vergleichsbeispiel 17

Zum Vergleich mit den unter Verwendung der erfindungsgemässen Bindemitteln erzielten Ergebnisse enthält das Bindemittel dieses Vergleichsbeispiels kein Aminosaccharid, sondern stattdessen als gebräuchlichen Zusatz ein Hexit. Das verwendete Bindemittel weist die folgende Zusammensetzung auf
90 Gew.-% Natronwasserglas analog Beispiel 1
10 Gew.-% einer 70 Gew.-%igen wässrigen Lösung von Hexit ($C_6H_8(OH)_6$).
Die Herstellung der Formkörper erfolgte analog Beispiel 1; die Ergebnisse sind gleichfalls in Tabelle 1 enthalten.

## Erläuterungen zu Tabelle 1

Es bedeuten:

a  =  Anfangswert der Druckfestigkeit
b  =  Druckfestigkeit nach dreitägiger Lagerung bei Raumtemperatur
c  =  Druckfestigkeit nach dreitägiger Lagerung bei Raumtemperatur und anschliessendem Tempern (900°C, 2 Minuten)

++ = Kantenfestigkeit sehr gut    (+) = Kantenfestigkeit befriedigend
+  = Kantenfestigkeit gut     — = Kantenfestigkeit schlecht

Tabelle 1

| Beispiel | organ. Substanz im Bindemittel (Gew.-%) | Druckfestigkeit (bar) | | | Hygroskopizität | Kantenfestigkeit | |
|---|---|---|---|---|---|---|---|
| | | a | b | c | | a | b |
| 1 | 3 | 5,4 | 70 | 4,1 | 0,93 | (+) | ++ |
| 2 | 3 | 8,4 | 66 | 3,5 | 0,87 | (+) | ++ |
| 3 | 3 | 7,8 | 67 | 5,3 | 1,29 | (+) | ++ |
| 4 | 3 | 5,1 | 69 | 8,5 | 1,42 | (+) | ++ |
| 5 | 3 | 6,2 | 30 | 7,0 | 0,87 | (+) | + |
| 6 | 3 | 11,0 | 58 | 4,6 | 1,34 | (+) | ++ |
| 7 | 3 | 9,1 | 64 | 4,3 | 1,39 | (+) | ++ |
| 8 | 3 | 6,3 | 56 | 5,5 | 1,37 | (+) | ++ |
| 9 | 3 | 9,2 | 46 | 5,7 | 1,33 | (+) | ++ |
| 10 | 0,1 | 3,8 | 84 | 8,8 | 1,20 | (+) | ++ |
| 11 | 5 | 5,9 | 50 | 9,0 | 1,31 | (+) | ++ |
| 12 | 10 | 9,9 | 32 | 3,8 | 1,29 | (+) | + |
| 13 | 3 | 6,5 | 61 | 8,8 | 1,32 | (+) | ++ |
| 14 | 0,5 | 4,0 | 76 | 5,8 | 0,95 | — | (+) |
| 15 | 3 | 7,0 | 58 | 4,8 | 0,78 | (+) | + |
| 16 | 3 | 9,4 | 42 | 3,4 | 0,67 | (+) | + |
| 17 | 7 | 5,6 | 44 | 18,0 | 1,76 | (+) | + |

Die in der Tabelle aufgelisteten Werte zeigen, dass — bezogen auf den Vergleichsversuch 17 — durch die Anwendung der erfindungsgemässen Bindemittel die Hygroskopizität der gefertigten Prüfkörper erheblich gesenkt, die Druckfestigkeit der Prüfkörper während der Lagerung hingegen wesentlich gesteigert werden kann. Von entscheidendem Vorteil ist ferner das verbesserte Zerfallverhalten nach Erhitzen der Prüfkörper; die nach dem Tempern ermittelten Druckfestigkeiten betragen 25 bis 50% des Vergleichswertes. Die mit den erfindungsgemässen Bindemitteln erzielbaren Vorteile lassen sich bereits mit wesentlich geringeren Konzentrationen an organischer Substanz im Bindemittel erreichen. Ein Zusatz von Kaliummetaborat zum Bindemittel bedingt auch bei verkürzter Begasungsdauer verbesserte Eigenschaften der Prüfkörper.

## Patentansprüche

1. Bindemittel auf der Basis wässriger Alkalimetallsilikatlösungen, die ein Molverhältnis $SiO_2:Me_2O$ im Bereich von 2,0 bis 3,4 : 1, wobei Me ein Alkalimetall bedeutet, und einen Feststoffgehalt von 35 bis 50 Gewichtsprozent aufweisen, dadurch gekennzeichnet, dass sie reduktiv aminierte Mono-, Di- oder Oligosaccharide enthalten.

2. Bindemittel nach Anspruch 1, dadurch gekennzeichnet, dass sie ein Alkalimetallsilikat mit einem Molverhältnis $SiO_2:Me_2O$ im Bereich von 2,2 bis 2,6 : 1 enthalten, wobei Me Natrium und/oder Kalium bedeutet.

3. Bindemittel nach Anspruch 1 und 2, dadurch gekennzeichnet, dass sie 0,1 bis 10 Gewichtsprozent, vorzugsweise 0,5 bis 5 Gewichtsprozent, bezogen auf die gesamte Bindemittelzusammensetzung, eines reduktiv aminierten Mono-, Di- oder Oligosaccharids enthalten.

4. Bindemittel nach Anspruch 1 bis 3, dadurch gekennzeichnet, dass sie als reduktiv aminiertes Mono-, Di- oder Oligosaccharid ein unter Einwirkung von Wasserstoff erhaltenes Umsetzungsprodukt von Glucose, Galaktose oder Maltose, beziehungsweise deren Gemischen, mit Methylamin, Ethylamin, Butylamin oder Ethanolamin, beziehungsweise deren Gemischen, enthalten.

5. Bindemittel nach Anspruch 1 bis 4, dadurch gekennzeichnet, dass sie 0,1 bis 2 Gewichtsprozent $B_2O_3$, bezogen auf die gesamte Bindemittelzusammensetzung, in Form von Kaliummetaborat enthalten.

6. Verwendung eines Bindemittels nach Anspruch 1 bis 5 zur Herstellung von Formkörpern aus feinteiligen Füllstoffen, insbesondere von Formen und/oder Kernen für den Metallguss, unter Zusatz gebräuchlicher anorganischer und/oder organischer Härter.

## Claims

1. Binders based on aqueous alkali metal silicate solutions in which the molar ratio of $SiO_2$ to $Me_2O$ (Me = alkali metal) is in the range from 2.0 to 3.4 : 1 and which have a solids content of from 35 to 50% by weight, characterized in that they contain reductively aminated mono-, di- or oligosaccharides.

2. Binders as claimed in claim 1, characterized in that they contain an alkali metal silicate in a molar ratio of $SiO_2$ to $Me_2O$ of from 2.2 to 2.6 : 1, Me representing sodium and/or potassium.

3. Binders as claimed in claims 1 and 2, characterized in that they contain from 0.1 to 10% by weight and preferably from 0.5 to 5% by weight, based on the overall composition of the binder, of a reductively aminated mono-, di- or oligosaccharide.

4. Binders as claimed in claims 1 to 3, characterized in that they contain as reductively aminated mono-, di- or oligosaccharide a product of the reaction in the presence of hydrogen of glucose, galactose or maltose, or mixtures thereof, with methylamine, ethylamine, butylamine or ethanolamine or mixtures thereof.

5. Binders as claimed in claims 1 to 4, characterized in that they contain from 0.1 to 2% by weight of $B_2O_3$ in the form of potassium metaborate, based on the overall composition of the binder.

6. The use of a binder of the type claimed in claims 1 to 5 for the production of shaped structures of finely particulate fillers, particularly metal-casting moulds and/or cores, using conventional inorganic and/or organic hardeners.

### Revendications

1. Liants à base de solutions aqueuses de silicate de métal alcalin qui présentent un rapport molaire $SiO_2:Me_2O$ dans l'intervalle de 2,0 à 3,4 : 1, Me signifiant un métal alcalin, et une teneur en matière solide de 35 à 50% en poids, caractérisés en ce qu'ils contiennent des mono-, di- ou oligosaccharides aminés par réduction.

2. Liants selon la revendication 1, caractérisés en ce qu'ils contiennent un silicate de métal alcalin ayant un rapport molaire $SiO_2:Me_2O$ dans l'intervalle de 2,2 à 2,6 : 1, Me signifiant du sodium et/ou du potassium.

3. Liants selon les revendications 1 et 2, caractérisés en ce qu'ils contiennent 0,1 à 10% en poids, de préférence 0,5 à 5% en poids, par rapport à la composition totale du liant, d'un mono-, di- ou oligosaccharide aminé par réduction.

4. Liants selon les revendications 1 à 3, caractérisés en ce qu'ils contiennent en tant que mono-, di- ou oligosaccharide aminé par réduction un produit de réaction, obtenu sous l'action d'hydrogène, de glucose, galactose ou maltose ou de leurs mélanges avec de la méthylamine, éthylamine, butylamine ou éthanolamine ou leurs mélanges.

5. Liants selon les revendications 1 à 4, caractérisés en ce qu'ils contiennent 0,1 à 2% en poids de $B_2O_3$ par rapport à la composition totale du liant, sous forme de métaborate de potassium.

6. Utilisation d'un liant selon les revendications 1 à 5 pour la fabrication de corps moulés à partir de matières de charge finement divisées, en particulier de moules et/ou de noyaux pour fonte métallique, avec addition des durcissants usuels minéraux et/ou organiques.